# EUROPEAN PATENT APPLICATION

(11) **EP 1 342 727 A1**
(43) Date of publication of application: **10.09.2003**
(21) Application number: 01983805.1
(22) Date of filing: 15.11.2001
(51) Int. Cl.: C07K 5/10, C07K 7/06, C07K 7/08, C07K 14/005, C07K 16/08, G01N 33/53

(54) **ANTIBODY AGAINST NORWALK VIRUS AND METHOD OF DETECTING VIRUS BY USING THE ANTIBODY**

(30) Priority: 15.11.2000 JP 2000348125
(71) Applicant: BML, Inc., Tokyo 151-0051 (JP)
(72) Inventor: KAGEYAMA, Tsutomu, General Laboratory, BML, Inc., Kawagoe-shi, Saitama 350-1101 (JP); KOJIMA, Shigeyuki, Kawagoe-shi, Saitama 350-1101 (JP); FUKUSHI, Shuetsu, Kawagoe-shi, Saitama 350-1101 (JP); HOSHINO, Fuminori, Kawagoe-shi, Saitama 350-1101 (JP); KATAYAMA, Kazuhiko, Murayama Branch Office, Musashimurayama-shi, Tokyo 208-0011 (JP)
(74) Representative: Bohmann, Armin K., Dr.
(86) International application number: JP0109980
(87) International publication number: WO02040509

(57) **Abstract**

Immunogens containing antigen peptides specific for Norwalk virus which are selected from the amino acid sequences represented by SEQ ID NOs: 1 to 4, etc. and Norwalk-virus-specific antibodies against the immunogens are constructed. By detecting a virus with the use of such virus-specific antibodies, Norwalk virus can be conveniently and accurately detected.

SEQUENCE LISTING

<110> BML, INC.

<120> Antibody against Norwalk Virus and Method for Detecting
      Norwalk Virus using the Antibody

<130> PBM58/PCT

<140>
<141>

<150> JP P2000-348125
<151> 2000-11-15

<160> 25

<170> PatentIn Ver. 2.1

<210> 1
<211> 11
<212> PRT
<213> Norwalk virus

<400> 1
Gln Gly Glu Phe Thr Ile Ser Pro Asn Asn Thr
  1               5                  10

<210> 2
<211> 12
<212> PRT
<213> Norwalk virus

<400> 2
Ser Arg Phe Tyr Gln Leu Lys Pro Val Gly Thr Ala
  1               5                  10

<210> 3
<211> 9

<212> PRT
<213> Norwalk virus

<400> 3
Gly Glu Phe Thr Val Ser Pro Arg Asn
1               5

<210> 4
<211> 11
<212> PRT
<213> Norwalk virus

<400> 4
Val Phe Thr Val Ser Cys Arg Val Leu Thr Arg
1               5                   10

<210> 5
<211> 8
<212> PRT
<213> Norwalk virus

<400> 5
Leu Ala Thr Ala Gly Gln Val Asn
1               5

<210> 6
<211> 5
<212> PRT
<213> Norwalk virus

<400> 6
Ile Asp Pro Trp Ile
1               5

<210> 7
<211> 14
<212> PRT
<213> Norwalk virus

<400> 7
Pro Gln Gly Glu Phe Thr Ile Ser Pro Asn Asn Thr Pro Gly
1               5                   10

<210> 8
<211> 9
<212> PRT
<213> Norwalk virus

<400> 8
Leu Gly Pro His Leu Asn Pro Phe Leu
1               5

<210> 9
<211> 5
<212> PRT
<213> Norwalk virus

<400> 9
Gln Met Tyr Asn Gly
1               5

<210> 10
<211> 7
<212> PRT
<213> Norwalk virus

<400> 10
Pro Leu Glu Asp Val Arg Asn
1               5

<210> 11
<211> 6
<212> PRT
<213> Norwalk virus

<400> 11
Met Leu Tyr Thr Pro Leu
1               5

<210> 12
<211> 7
<212> PRT
<213> Norwalk virus

<400> 12
Phe Leu Phe Leu Val Pro Pro
1               5

<210> 13
<211> 5
<212> PRT
<213> Norwalk virus

<400> 13
Leu Ser Asn Ser Arg
1               5

<210> 14
<211> 7
<212> PRT
<213> Norwalk virus

<400> 14
Val Gln Phe Gln Asn Gly Arg
1               5

<210> 15
<211> 5
<212> PRT
<213> Norwalk virus

<400> 15
Leu Gly Glu Phe Lys
1               5

<210> 16
<211> 5

<212> PRT
<213> Norwalk virus

<400> 16
Thr Cys Val Pro Asn
1               5

<210> 17
<211> 15
<212> PRT
<213> Norwalk virus

<400> 17
Ser Trp Val Ser Arg Phe Tyr Gln Leu Lys Pro Val Gly Thr Ala
1               5                   10                  15

<210> 18
<211> 6
<212> PRT
<213> Norwalk virus

<400> 18
Asn Phe Val Gln Ala Pro
1               5

<210> 19
<211> 8
<212> PRT
<213> Norwalk virus

<400> 19
Leu Ala Gly Asn Ala Phe Thr Ala
1               5

<210> 20
<211> 8
<212> PRT
<213> Norwalk virus

<400> 20
Ala Met Leu Tyr Thr Pro Leu Arg
1               5

<210> 21
<211> 13
<212> PRT
<213> Norwalk virus

<400> 21
Asp Val Phe Thr Val Ser Cys Arg Val Leu Thr Arg Pro
1               5                   10

<210> 22
<211> 6
<212> PRT
<213> Norwalk virus

<400> 22
Ser Asn Ser Arg Phe Pro
1               5

<210> 23
<211> 5
<212> PRT
<213> Norwalk virus

<400> 23
Leu Phe Phe Arg Ser
1               5

<210> 24
<211> 6
<212> PRT
<213> Norwalk virus

<400> 24
Asn Pro Asp Thr Gly Arg
1               5

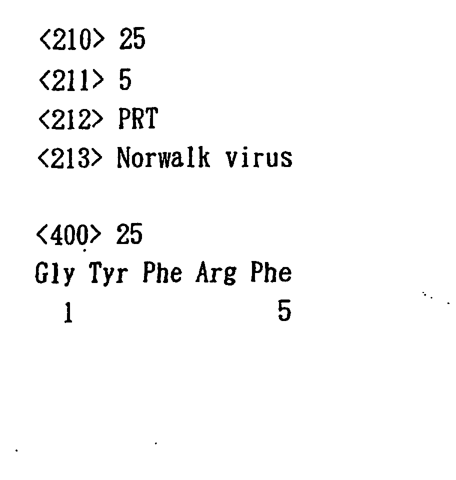

## Description

### Technical Field

The present invention relates to a method of detecting a virus.

### Background Art

The term "food poisoning" generally brings to mind bacterial food poisoning caused by bacteria such as *Salmonella, Vibrio parahaemolyticus*, and pathogenic *E. coli*, or natural toxin food poisoning caused by natural toxins contained in, for example, globefish or mushrooms. In addition, a very large number of food poisoning cases are caused by viruses, such as Norwalk virus, rotavirus, astrovirus, enterovirus, and adenovirus. Recent epidemiological research has revealed that, among other viruses, NVs are typical food-poisoning viruses.

Norwalk virus is a virus which was first identified in 1972 as the Norwalk virus (strain name) after an outbreak of gastrointestinal illness in the U.S.A. Under an electron microscope, the virus is observed as a small spherical virus of about 30 nm in diameter having an unclear surface structure, and since then viruses having similar shapes have been collectively called "small round structured viruses" (SRSVs); However, at present, the term SRSV is considered not to be used, for the reasons described hereinlater. In the meantime, in 1974, calicivirus, which had been well known in veterinary medicine and which measures about 30 nm in diameter and assumes a typical surface structure resembling a "Star of David," was first identified in a human patient; specifically, in a patient suffering winter vomiting disease, which was at that time epidemic in Britain. Since then, viruses having a shape similar to the above have been called classical human caliciviruses. These viruses are very difficult to grow in tissue culture cells or in experimental animals, and therefore, for some time the only feasible method was to analyze and culture the viruses on volunteers by use of stool specimens. Thus, characterization of the viruses was quite difficult. In 1990, a research group led by X. Jiang cloned the genome of Norwalk virus (strain name), and since then, gene analysis of these viruses has seen progress, revealing that an SRSV and a classical human calicivirus both belong to the family of *Caliciviridae,* having a single stranded "plus" RNA (plus-stranded). In 1999, the International Committee on Taxonomy of Viruses formally decided to abandon use of the names "SRSV" and "human calicivirus" and authorized use of the species names of these viruses; i.e., "Norwalk virus" and "Sapporo virus (SV). Therefore, in the present specification, the species name "Norwalk virus" is employed, and the term NV may also be used. In this connection, it should be noted that the genus-based names "Norwalk-like viruses" and "Sapporo-like viruses" are interim names. From an accumulation of data of genomic nucleotide sequences of viruses collected from a vast number of clinical specimens, NVs have been confirmed to be classified into two genogroups I and II; i.e., genogroup I (G1) (also described as Norwalk virus (G1), NV(G1), or (G1)) encompassing Norwalk viruses (strain names) and Southampton viruses among other viruses; and genogroup II (also described as Norwalk virus (G2), NV (G2), or (G2)) encompassing Hawaii viruses, Snow Mountain viruses, and similar viruses.

Infection with NV induces severe vomiting, diarrhea, and enterogastritis.

Conceivable sources of infection with NV are shellfishes such as oysters and mussels; and environmental water such as drinking water and sea water. In a mass outbreak of food-poisoning from sandwiches that occurred in the U.S.A in 1998, sliced ham for preparing the sandwiches was found to have been contaminated with NV. Although one of the workers who handled the ham did not have diarrhea, her child (infant) developed severe diarrhea, thus contagion of NV contamination via the worker from stool of her child was suspected. As in this case, NV is considered to be carried directly or indirectly from the stool or vomitus of an infected subject, even when the number of viruses is very small. Presumably, NVs excreted in stool or vomitus are transferred to the sea through sewage water or river water, then are ingested by shellfishes, for example, oysters, living in that sea area, and when humans ingest such shellfishes, infection to humans occurs again, thereby constituting an infection cycle.

Identification of the cause and the contamination source is a critical issue. That is, the food-poisoning patients must be treated as quickly as possible through appropriate selection of a therapeutic method, which would be realized by identifying the cause of the food poisoning, and simultaneously, spreading of food poisoning must be stopped by identifying the contamination source as early as possible. Means which has so far been employed for identifying the cause of NV contamination is searching for viral particles under an electron microscope, and thus, identification has required significant labor and time. Specifically, electron microscopy requires a large facility, which means only a limited number of institutions can perform detection tests. In addition, virus detection operation *per se* is cumbersome and thus disadvantageous. What is more, detection of viruses through electron microscopy from foodstuffs, kitchen utensils, or similar objects which are potentially responsible for viral food poisoning is extremely difficult, because of insufficient sensitivity.

Meanwhile, detection of NV through RT-PCR, which is considered to provide detection efficiency higher than that achievable by electron microscopy, cannot be said to be an optimal means for detecting NV, because the nucleotide sequence of NV genes has high divergence and thus consensus primer sequences have not yet been found. In addition, the following shortcomings are noted: In general, the detection procedure of RT-PCR is intricate; pseudo-positive results may result when the experimental system is contaminated with the PCR product; and many reagents employed in the reaction, such as enzymes, are expensive.

Accordingly, an object of the present invention is to provide convenient and accurate means for detecting NV.

### Disclosure of the Invention

The present inventors have considered that if an antibody which is specific to NV can be created, use of such a specific antibody might enable construction of a simple and accurate system for detecting NV. However, creating a specific antibody of interest requires discovery of an antigen which induces production of antibodies which can bind to a broad range of numerous NV variants. As described above, since NV genes have diversified nucleotide sequences, it is not an easy task to find out a common antigen of NV variants.

NV is a virus which belongs to the *Caliciviridae* family and consists of a linear (+)stranded RNA genome. The genomic RNA contains three ORFs (open reading frames) in a fragment of about 7.7 kb (which bears a poly(A) portion) at its 3' end. ORF1 at the 5' end of NV genomic RNA bears coding regions for coding a variety of nonstructural proteins such as viral-replication-related RNA-dependent RNA polymerase, and ORF2 at the 3' end of NV genomic RNA bears coding regions for coding structural proteins (the functions of ORF3 are yet to be elucidated). Of the two ORFs, ORF2, which codes for capsid protein (note: the capsid protein is considered to form the outer shell of the virus), is of high divergence and presumably this divergence results in diversified NVs having different serotypes.

Thus far, many types of antibodies have already been obtained as antibodies corresponding to such respective serotypes, and a common antibody that reacts with any and all species of NV type or a common antibody that reacts with NV(G1) or NV(G2) has not yet been obtained.

The present inventors have carried out detailed analyses using many NV genes obtained from stool specimens of a great number of NV-infected patients, and as a result, have found that a highly conserved region is contained in a gene region coding for capsid protein, although such a region is usually diversified.

The present inventors have now discovered the below-listed four peptides having the amino acid sequences shown below are encoded by highly conserved gene regions and are suitably employed as antigens for producing antibodies against NV.
1) Gln-Gly-Glu-Phe-Thr-Ile-Ser-Pro-Asn-Asn-Thr (SEQ ID NO: 1)
2) Ser-Arg-Phe-Tyr-Gln-Leu-Lys-Pro-Val-Gly-Thr-Ala (SEQ ID NO: 2)
3) Gly-Glu-Phe-Thr-Val-Ser-Pro-Arg-Asn (SEQ ID NO: 3)
4) Val-Phe-Thr-Val-Ser-Cys-Arg-Val-Leu-Thr-Arg (SEQ ID NO: 4)

Of the above peptides, the peptides 1) and 2), having the amino acid sequences of SEQ ID NOs: 1 and 2, are capable of imparting NV(G1) binding ability (that is, ability to bind to NV(G1), but no ability to bind to NV(G2)) to antibodies which take the peptides 1) and 2) as their antigens. Also, when antibodies which take the peptides 3) and 4), having the amino acid sequences of SEQ ID NOs: 3 and 4, as their antigens are polyclonal antibodies, these peptides 3) and 4) are capable of imparting both NV(G1) binding ability and NV(G2) binding ability to the polyclonal antibodies, whereas when the antibodies are monoclonal antibodies, these peptides 3) and 4) are capable of imparting NV(G2) binding ability (that is, ability to bind to NV(G2), but no ability to bind to NV(G1)) to the monoclonal antibodies.

Accordingly, in the present application, the present inventors provide the following.

A first invention is drawn to immunogens each containing an antigen peptide composed of at least 4 continuous amino acid residues that fall within any of the amino acid sequences of SEQ ID NOs: 1 to 4 (hereinafter the immunogens may be referred to as the present immunogen or present immunogens).

A second invention is drawn to antibodies which take as antigens immunogens each containing an antigen peptide composed of at least 4 continuous amino acid residues that fall within either one of the amino acid sequences of SEQ ID NOs: 1 and 2, and each being capable of binding to NV(G1) but not binding to NV(G2).

A third invention is drawn to antibodies which take as antigens immunogens each containing an antigen peptide composed of at least 4 continuous amino acid residues that fall within either one of the amino acid sequences of SEQ ID NOs: 3 and 4, and each being capable of binding to NV(G1) and NV(G2).

A fourth invention is drawn to monoclonal antibodies which take as antigens immunogens each containing an antigen peptide composed of at least 4 continuous amino acid residues that fall within either one of the amino acid sequences of SEQ ID NOs: 3 and 4, and each being capable of binding to NV(G2) but not binding to NV(G1) (hereinafter the antibodies mentioned in relation to the second to the fourth inventions immunogens may be collectively referred to as the present antibody or the present antibodies).

A fifth invention is drawn to a method of detecting a virus by use of the present antibody, in which NV present in a specimen is detected as an NV(G1), an NV(G2), or as a combination of an NV(G1) and an NV(G2) (hereinafter the method may be referred to as the present detection method).

A sixth invention is drawn to a virus detection kit for performing the present detection method, the kit containing the present antibody as an element thereof (hereinafter the kit may be referred to as the present kit).

### Brief Description of the Drawings

Fig. 1 is a photograph showing CBB stains obtained from electrophoresis performed on respective VLPs (virus-like particles);
Fig. 2 is a photograph showing Western blots obtained in the study of bonding ability of the present antibody 1A to the respective VLPs;
Fig. 3 is a photograph showing Western blots obtained in the study of bonding ability of the present antibody 1B to the respective VLPs;
Fig. 4 is a photograph showing Western blots obtained in the study of bonding ability of the present antibody 2A to the respective VLPs;
Fig. 5 is a photograph showing Western blots obtained in the study of bonding ability of the present antibody 2B to the respective VLPs; and
Fig. 6 is a photograph showing Western blots obtained in the study of bonding ability of the present antibody 3A to the respective VLPs.

### Best Mode for Carrying Out the Invention

Modes for carrying out the present invention will next be described.

### 1. The present immunogen

Antigen peptides having amino acid sequences of SEQ ID NOs: 1 to 4 and contained in the present immunogens may be synthesized through a well-known peptide synthesis method. Specifically, antigen peptides of interest may be synthesized through, for example, the sequential elongation method, the fragment condensation method, the solid-phase method (e.g., the F-moc method), or the liquid-phase method. Needless to say, the antigen peptides may alternatively be synthesized with a peptide synthesizer. Moreover, each of the antigen peptides may also be produced through a process in which a nucleic acid coding for an antigen peptide of interest is synthesized, the resultant nucleic acid is integrated into a gene of a host (e.g., *E. coli*), and the recombinant is subjected to a genetic engineering method for collecting antigen peptides, to thereby yield the antigen peptide of interest.

The minimum number of continuous amino acid residues in an antigen peptide is determined to be 4, because if the number of amino acid residues is less than 4, a segment corresponding to the amino acid sequence is highly likely to be present in viruses or microorganisms other than NV, leading to unacceptably large detection error in the present detection method. The maximum number of amino acid residues of each of the antigen peptides of SEQ ID NOs: 1 to 4 is the number of the amino acid residues that make up the entirety of the peptide. Specifically, such maximum numbers of amino acid residues are 11 for SEQ ID NO: 1, 12 for SEQ ID NO: 2, 9 for SEQ ID NO: 3, and 11 for SEQ ID NO: 4.

The present immunogens may be the above-described antigen peptides *per se,* or may be optionally modified species of the above-described antigen peptides. Exemplary modification is modification with a hapten. In general, immunization with a low-molecular-weight peptide alone, such as any one of the above-described antigen peptides, does not result in successful production of antibodies, and therefore, the present immunogens are preferably transformed into complexes which are prepared by adding haptens to the mentioned antigen peptides. Examples of haptens which may be used with the present immunogens include keyhole limpet hemocyanin (KLH), bovine serum albumin (BSA), ovalbumin, and other substances which are widely used as haptens. Addition of these haptens to antigen peptides may be carried out through any known conjugation method, such as the gultaraldehyde method, the carbodiimide method, or the maleimide method. Also, in accordance with the selected conjugation method, antigen peptides may be subjected to necessary treatment; for example, in the case where the maleimide method is used employing MBS (m-maleimidobenzoyl-N-hydroxy-succinimide ester), a cysteine residue may be added to either end of the antigen peptide so as to attain disulfide bonding during condensation reaction.

Moreover, as will be described hereinbelow, transformants which have been obtained through transformation by use of a gene coding for an antigen peptide may serve as immunogens. Such transformants also fall within the technical scope of the immunogens of the present invention. Furthermore, gene expression vectors which harbor a gene coding for an antigen peptide and which can be produced through direct expression of an immunogen in an immunized animal also fall within the technical scope of the immunogens of the present invention.

Thus, there can be produced an immunogen of the present invention, which contains an antigen peptide of interest composed of at least 4 continuous amino acid residues that fall within any of the amino acid sequences of SEQ ID NOs: 1 to 4.

In addition to the above-described antigen peptides, the following peptides may be exemplified as potential antigen peptides capable of producing NV specific immunological antibodies.

Firstly, antigen peptides capable of producing NV(G1) antibodies include:
Antigen peptides composed of 4 to 8 continuous amino acid residues that fall within the amino acid sequence of Leu-Ala-Thr-Ala-Gly-Gln-Val-Asn (SEQ ID NO: 5).
Antigen peptides composed of 4 or 5 continuous amino acid residues that fall within the amino acid sequence of Ile-Asp-Pro-Trp-Ile (SEQ ID NO: 6).
Antigen peptides composed of 4 to 14 continuous amino acid residues that fall within the amino acid sequence of Pro-Gln-Gly-Glu-Phe-Thr-Ile-Ser-Pro-Asn-Asn-Thr-Pro-Gly (SEQ ID NO: 7).
Antigen peptides composed of 4 to 9 continuous amino acid residues that fall within the amino acid sequence of Leu-Gly-Pro-His-Leu-Asn-Pro-Phe-Leu (SEQ ID NO: 8).
· Antigen peptides composed of 4 or 5 continuous amino acid residues that fall within the amino acid sequence of Gln-Met-Tyr-Asn-Gly (SEQ ID NO: 9).
· Antigen peptides composed of 4 to 7 continuous amino acid residues that fall within the amino acid sequence of Pro-Leu-Glu-Asp-Val-Arg-Asn (SEQ ID NO: 10).
· Antigen peptides composed of 4 to 6 continuous amino acid residues that fall within the amino acid sequence of Met-Leu-Tyr-Thr-Pro-Leu (SEQ ID NO: 11).
· Antigen peptides composed of 4 to 7 continuous amino acid residues that fall within the amino acid sequence of Phe-Leu-Phe-Leu-Val-Pro-Pro (SEQ ID NO: 12).
· Antigen peptides composed of 4 or 5 continuous amino acid residues that fall within the amino acid sequence of Leu-Ser-Asn-Ser-Arg (SEQ ID NO: 13).
· Antigen peptides composed of 4 to 7 continuous amino acid residues that fall within the amino acid sequence of Val-Gln-Phe-Gln-Asn-Gly-Arg (SEQ ID NO: 14).
· Antigen peptides composed of 4 or 5 continuous amino acid residues that fall within the amino acid sequence of Leu-Gly-Glu-Phe-Lys (SEQ ID NO: 15).
· Antigen peptides composed of 4 or 5 continuous amino acid residues that fall within the amino acid sequence of Thr-Cys-Val-Pro-Asn (SEQ ID NO: 16).
· Antigen peptides composed of 4 to 15 continuous amino acid residues that fall within the amino acid sequence of Ser-Trp-Val-Ser-Arg-Phe-Tyr-Gln-Leu-Lys-Pro-Val-Gly-Thr-Ala (SEQ ID NO: 17).

Secondly, antigen peptides capable of producing NV(G2) antibodies include:
· Antigen peptides composed of 4 to 6 continuous amino acid residues that fall within the amino acid sequence of Asn-Phe-Val-Gln-Ala-Pro (SEQ ID NO: 18).
· Antigen peptides composed of 4 to 8 continuous amino acid residues that fall within the amino acid sequence of Leu-Ala-Gly-Asn-Ala-Phe-Thr-Ala (SEQ ID NO: 19).
· Antigen peptides composed of 4 to 8 continuous amino acid residues that fall within the amino acid sequence of Ala-Met-Leu-Tyr-Thr-Pro-Leu-Arg (SEQ ID NO: 20).
· Antigen peptides composed of 4 to 13 continuous amino acid residues that fall within the amino acid sequence of Asp-Val-Phe-Thr-Val-Ser-Cys-Arg-Val-Leu-Thr-Arg-Pro (SEQ ID NO: 21).
· Antigen peptides composed of 4 to 6 continuous amino acid residues that fall within the amino acid sequence of Ser-Asn-Ser-Arg-Phe-Pro (SEQ ID NO: 22).
· Antigen peptides composed of 4 or 5 continuous amino acid residues that fall within the amino acid sequence of Leu-Phe-Phe-Arg-Ser (SEQ ID NO: 23).
· Antigen peptides composed of 4 to 6 continuous amino acid residues that fall within the amino acid sequence of Asn-Pro-Asp-Thr-Gly-Arg (SEQ ID NO: 24).
· Antigen peptides composed of 4 or 5 continuous amino acid residues that fall within the amino acid sequence of Gly-Tyr-Phe-Arg-Phe (SEQ ID NO: 25).

### 2. The present antibodies

The present antibodies are polyclonal or monoclonal antibodies which are produced through immunization of an animal with an immunogen of the present invention which comprises an antigen peptide composed of at least 4 continuous amino acid residues that fall within any of the amino acid sequences of SEQ ID NOs: 1 to 4. As used herein, unless otherwise specified, the term "antibody" or "antibodies" refer to both polyclonal and monoclonal antibodies.

Immunization of animals with the present immunogens may be performed through a customary procedure. No particular limitations are imposed on the animals, and mammals such as rabbits, goat, rats, and mice may be used. Each of the present immunogens is suspended in saline or in a buffer which is generally employed in physiological tests or similar tests, or is mixed with an adjuvant such as Freund's adjuvant, and then administered to an animal for immunization via an appropriate route; e.g., intraperitoneally, intramuscularly, intravenously, percutaneously, or subcutaneously. The dose of the present immunogens depends on, for example, the administration route, the species of the mammal, and other factors. For example, in the case of subcutaneous administration to a rabbit, a dose of about 0.2 mg/rabbit is preferred. Generally, three or more injections of the present immunogen performed at intervals of 2 to 14 days will provide the present antibody having sufficient potency in the form of a polyclonal antibody from serum of the immunized animal.

Alternatively, the present antibody in the form of a polyclonal antibody may be prepared through the following process: Cells derived from an animal of the same species and lineage as those of an animal to be immunized are transformed through insertion, to the cells, of an expression vector which harbors a gene coding for an antigen peptide composed of at least 4 continuous amino acid residues that fall within any of the amino acid sequences of SEQ ID NOs: 1 to 4. The resultant transformants are transplanted into the animal for immunization, whereby polyclonal antibodies of interest can be prepared. In other words, within the body of the animal to which the transformants had been transplanted, the transformants continuously produce the above-described antigen peptide, etc., prompting production of antibodies against the antigen peptide, and thus, such antibodies serve as the present antibody (Nemoto, T., *et al.,* Eur. J. Immunol., 25, 3001 (1995)).

Production of the present antibody in the form of a monoclonal antibody can be attained through a known method disclosed by, for example, Kohler and Milstein (Kohler, G. and Milstein, C., Nature, 256, 495 (1975)). For example, immunocytes are collected from the spleen of the immunized animal, and the immunocytes and, for example, myeloma cells are subjected to cell fusion in the presence of a fusion promoter such as polyethylene glycol, in a conventional culture medium to which, if desired, an auxiliary agent such as dimethyl sulfoxide is added for the purpose of improving fusion efficiency, to thereby prepare hybridomas. Myeloma cells which have already been known may be employed, and examples thereof include SP2/0-Ag14, P3-NS1-1-Ag4-1, MPC11-45,6.TG.7 (derived from a mouse); 210.RCY.Ag1.2.3 (derived from a rat); and SKO-007, GM15006TG-A12 (derived from a human).

When the present antibodies are monoclonal antibodies, strains capable of producing such monoclonal antibodies can be chosen by application, to antibodies produced by hybridomas, of a customary retrieval method such as ELISA, the plaque method, the spot method, the agglutination method, the Ouchterlony method, or RIA. Recovery of monoclonal antibodies from the thus-obtained monoclonal antibody producing strains may be performed in accordance with a routine method. For example, antibodies can be collected from the culture supernatant, or alternatively, a hybridoma is administered to an animal having compatibility with the hybridoma, multiplying the hybridoma, and monoclonal antibodies can be recovered from the ascites of the animal.

When the antigen peptide of interest is a peptide composed of at least 4 continuous amino acid residues that fall within either one of the amino acid sequences of SEQ ID NOs: 3 and 4, the monoclonal antibody which is prepared through the above-described procedure binds to NV(G2) at high incidences, but does not bind to NV(G1). (Note: Monoclonal antibodies other than this type are considered to bind to both NV(G1) and NV(G2).) Selection of the monoclonal antibody of interest can be carried out through a routine method. In particular, the monoclonal antibody produced by a hybridoma prepared through use, as an antigen, of a peptide composed of at least 4 continuous amino acid residues that fall within either one of the amino acid sequences of SEQ ID NOs: 3 and 4 is highly likely to be specific to NV(G2). Therefore, when monoclonal antibodies obtained from respective hybridomas are checked for their ability to bind to capsid protein derived from NV(G1) and/or NV(G2) in a one-by-one manner, selection of a NV(G2)-specific monoclonal antibody of interest can be achieved with ease (see Examples).

The present antibodies, which are polyclonal or monoclonal, are beneficially used after being concentrated and purified through a routine method; e.g., affinity chromatography employing a carrier which bears protein A or antigen peptide, salting out, or gel filtration.

The thus-obtained antibodies of the present invention may be labeled with a suitable labeling substance which is selected in accordance with the below-described present detection method. Needless to say, such labeled antibodies fall within the technical scope of the present invention.

Labeling substances to be employed in the present invention are those which *per se*, or upon reaction with other substances, provide detectable signals. Specific examples of the labeling substances include labeling enzymes such as horseradish peroxidase, alkaline phosphatase, β-D-galactosidase, glucose oxidase, glucose-6-phosphate dehydrogenase, alcohol dehydrogenase, malate dehydrogenase, penicillinase, catalase, apo-glucose oxidase, urease, and luciferase; fluorescent substances such as fluorescein isothiocyanate, phycobilin protein, rare earth metal chelates, dansyl chloride, tetramethylrhodamine isothiocyanate, green fluorescent protein (GFP), and red fluorescent protein (RFP); radioisotopes such as ¹²⁵I, ¹⁴C, and ³H; chemical substances such as biotin, avidin, and digoxigenin; or chemiluminescent substances. Labeling of an antibody with any of these labeling substances may be performed appropriately through use of a known labeling method in accordance with the type of the labeling substance.

Of the thus-obtained antibodies of the present invention, those produced against an immunogen containing an antigen peptide composed of at least 4 continuous amino acid residues that fall within either one of the amino acid sequences of SEQ ID NOs: 1 and 2 bind specifically to NV(G1); in other words, they bind to NV(G1) but do not bind to NV(G2).

In contrast, those produced against an immunogen containing an antigen peptide composed of at least 4 continuous amino acid residues that fall within either one of the amino acid sequences of SEQ ID NOs: 3 and 4 are classified into the following two categories: 1) When they are polyclonal antibodies, they bind to NV(G1) and NV(G2), whereas 2) when they are monoclonal antibodies, the incidence at which they specifically bind to NV(G2) is high; that is, they tend to bind to NV(G1) but do not bind to NV(G2).

### 3. The present detection method and the present kit

The present detection method is directed to a virus detection method for detecting NV, making use of the specificity of the present antibody against NV.

Specific modes of the present detection method include detection through immunoassays. Examples of immunoassays include those of competitive format, such as the first-antibody-immobilizing method, the two-antibody method, EMIT (enzyme multiplied immunoassay technique), enzyme-channeling immunoassay, immunoassay by use of a labeling substance for modifying enzyme activity, and liposome membrane - enzyme immunoassay; and those of non-competitive format such as the sandwich technique, the immunoenzymometry, enzyme activity-enhanced immunoassay, and proximal linkage immunoassay. In the present detection method employing these immunoassays, an antibody of the present invention, labeled or non-labeled, is used as a specific antibody in a step of antigen-antibody reaction between the assay object and a specific antibody therefor, to thereby obtain measurements regarding the target of measurement; i.e., NV. The measurements are correlated to the presence of NV, whereby NV in a specimen can be detected.

Depending on the assay technique employed in the present detection method, the present antibody and antigen protein/peptide may be used after having been immobilized onto an insoluble carrier so as to assume the form of immobilized antibody or immobilized antigen. Carriers for immobilization may assume the form of plate, ball, stick, test tube, bead, filter paper, or membrane, and the material therefor may be, for example, polystyrene, polycarbonate, polypropylene, or polyvinyl, which adsorbs protein/peptide or antibodies quite effectively. Moreover, examples of the carriers for immobilization include insoluble carriers such as a cellulose carrier, agarose carrier, polyacrylamide carrier, dextran carrier, poly(vinyl alcohol) carrier, polystyrene carrier, poly(amino acid) carrier, and porous silica carrier. The antibodies or antigen proteins/peptides of the present invention can be immobilized onto such a carrier for immobilization through a method known *per se.*

Specimens which may be employed in the present detection method encompass any and all types of objects in which the presence or absence of NV is critical. For example, the specimen may be biological components such as plasma, serum, and blood; excrement such as stool, vomitus, and sweat; water from watery sources which may be polluted with NV, such as river water, seawater, lake water, sewage, and various types of foul water; food; deposits collected from food production facilities; clothing of food production workers, etc. A specimen which undergoes the present detection method may be prepared by processing the above described objects with a method suited for the type of the specimen. Typically, the specimen is immersed or suspended in water, etc., to obtain supernatant, and then a fraction of the supernatant may be employed.

By selecting an antibody among other antibodies of the present invention, the present detection method enables detection of different NVs.

Specifically, when the present antibody employed is an antibody produced against an immunogen containing an antigen peptide composed of at least 4 continuous amino acid residues that fall within either one of the amino acid sequences of SEQ ID NOs: 1 and 2 and capable of binding to NV(G1) (hereinafter the antibody corresponding to the amino acid sequence of SEQ ID NO: 1 may be referred to as the present antibody 1A, the antibody corresponding to the amino acid sequence of SEQ ID NO: 2 may be referred to as the present antibody 1B, and these two may be collectively referred to as the present antibody 1; and the present detection method involving antibody 1 may be referred to as the present detection method 1), since the present antibody 1 binds only to NV(G1) and does not bind to NV(G2), the present detection method 1 can detect only NV(G1). Therefore, if this detection method provides a result of NV positive, at least the presence of NV(G1) in the specimen can be determined. However, presence or absence of NV(G2) cannot be verified on the basis of this information alone.

On the other hand, when the present antibody employed is an antibody (which may be monoclonal or polyclonal) produced against an immunogen containing an antigen peptide composed of at least 4 continuous amino acid residues that fall within either one of the amino acid sequences of SEQ ID NOs: 3 and 4 and capable of binding to both of NV(G1) and NV(G2) (hereinafter the antibody corresponding to the amino acid sequence of SEQ ID NO: 3 and capable of binding to NV(G1) and NV(G2) may be referred to as the present antibody 2A, the antibody corresponding to the amino acid sequence of SEQ ID NO: 4 and capable of binding to NV(G1) and NV(G2) may be referred to as the present antibody 2B, and these two may be collectively referred to as the present antibody 2; and the present detection method involving antibody 2 may be referred to as the present detection method 2), since the present antibody 2 binds to both NV(G1) and NV(G2), the present detection method 2 can detect NV. Therefore, if this detection method provides a result of NV positive, at least the presence of NV in the specimen is confirmed. However, this information alone cannot determine whether the NV is NV(G1) or NV(G2), or both NV(G1) and NV(G2).

Moreover, when the present antibody employed is an antibody (monoclonal antibody) produced against an immunogen containing an antigen peptide composed of at least 4 continuous amino acid residues that fall within either one of the amino acid sequences of SEQ ID NOs: 3 and 4 and capable of binding to NV(G2) (hereinafter the antibody corresponding to the amino acid sequence of SEQ ID NO: 3 and capable of binding specifically to NV(G2) may be referred to as the present antibody 3A, the antibody corresponding to the amino acid sequence of SEQ ID NO: 4 and capable of binding specifically to NV(G2) may be referred to as the present antibody 3B, and these two may be collectively referred to as the present antibody 3; and the present detection method involving antibody 3 may be referred to as the present detection method 3), since the present antibody 3 binds to NV(G2) but does not bind to NV(G1), the present detection method 3 can detect only NV(G2). Therefore, if this detection method provides a result of NV positive, at least the presence of NV(G2) in the specimen can be determined. However, presence or absence of NV(G1) cannot be verified on the basis of this information alone.

For example, when food poisoning occurs and urged needs are faced for identifying the cause and determining a proper therapeutic approach, such purposes can be attained if the present detection method can detect as to whether or not a virus responsible for the food poisoning is NV, because the therapeutic approach for therapy of poisoning caused by NV is essentially the same for (G1) and (G2). Thus, in such a case, use of the present detection method 2 alone can attain the purpose of the use of the present detection method(s).

However, if the contamination source is desired to be determined, use of the present detection method 2 alone is insufficient, because there may be cases where NV found in the contamination source does not coincide with NV from the patients of food-poisoning, and in such cases, there arises a need for at least determining as to whether or not the genotype of the NV from the contamination source is identical with that of the NV from the patients. In such a case, a possible approach is, for example, in the first step, the presence of NV is confirmed by use of the present detection method 2, and in the second step, the genotype of the detected NV is determined by use of the present detection methods 1 and 3 in combination. In other words, if specimens which have tested positive with the present detection method 2 undergo the present detection method 1 and the result therefrom is positive, the genotype of the NV is determined to be G1. Similarly, if specimens which have tested positive with the present detection method 2 undergo the present detection method 3 and the result therefrom is positive, the genotype of the NV is determined to be G2.

Possible cases where only the present detection method 1 is performed include cases where it is clear that the virus responsible for the prevalence is NV(G1).

Similarly, possible cases where only the present detection method 3 is performed include cases where it is clear that the virus responsible for the prevalence is NV(G2).

As described above, the present invention provides:
a virus detection method in which NV(G1) contained in a specimen is detected by use of the present antibody 1;
a virus detection method in which NV(G1) and/or NV(G2) contained in a specimen is detected by use of the present antibody 2;
a virus detection method in which NV(G2) contained in a specimen is detected by use of the present antibody 3; and
a virus detection method in which NV contained in a specimen is detected by use of two or more of the present antibodies 1 to 3 in combination.

The present kit is used for detecting a virus by carrying out the detection method of the present invention. Specific elements that constitute the kit may be appropriately determined in accordance with the assay format employed in the present detection method that is chosen. In any event, however, at least one of the present antibodies is contained.

Depending on the assay format employed in the detection method that is chosen, an antibody of the present invention included as an element of the present kit may be non-labeled or labeled. Also, the antibody serving as an element of the kit may be in the form of an immobilized antibody.

Other elements which may be included in the present kit include elements which are usually included in detection kits making use of antigen-antibody reaction, and examples thereof include a dilution buffer, a color developer solution corresponding to the color developing mechanism, a washing solution, reaction plates, reaction beads, reaction tubes, and a blocking solution (such as BSA or skim milk).

The present kit may be used such that each step of the present detection method described above employs a corresponding kit element or elements, so that efficient detection of NV by the present detection method can be attained.

As described above, the present invention provides:
a virus detection kit which contains the present antibody 1 as an element and which is used for carrying out the present detection method 1;
a virus detection kit which contains the present antibody 2 as an element and which is used for carrying out the present detection method 2;
a virus detection kit which contains, as an element, the present antibody 3 in the form of a monoclonal antibody and which is used for carrying out the present detection method 3; and
a virus detection kit which contains, as elements, 2 or more species of the present antibodies 1 to 3 and which is used for carrying out 2 or more methods of the present detection methods 1 to 3.

### Example

The present invention will next be described by way of example, which should not be construed as limiting the technical scope of the invention.

### Production of the present immunogen and the present antibody

### 1) Production of polyclonal antibody

Antigen peptides having amino acid sequences of SEQ ID NOs: 1 to 4 (hereinafter, each antigen peptide having an amino acid sequence of SEQ ID NO: 1, 2, 3, or 4 may be referred to as "antigen peptide 1, 2, 3, or 4," corresponding to its sequence number) were synthesized through a solid phase synthesis method (the F-moc solid phase method) employing a peptide synthesizer (SHIMADZU PSSM-8: Shimadzu Corporation). For conjugation with KLH through the maleimide method, cysteine was added to the C-terminus of the peptide having an amino acid sequence of SEQ ID NO: 2 or 3. Each of the crude peptides thus synthesized was purified by use of a reverse phase HPLC column (µBondsphere 5 µ C18 100 Å, manufactured by Waters Corporation, USA), to thereby yield a peptide having a purity of 80% or more. The purified antigen peptide 1 or 4 was conjugated with KLH by the glutaraldehyde method, and the purified antigen peptide 2 or 3 was conjugated with KLH by the maleimide method. Each of the four present immunogens thus obtained (500 µg), which had been obtained in the form of a complex between a hapten (KLH) and the antigen peptide 1, 2, 3, or 4, was mixed with complete Freund's adjuvant (500 µL), whereby an emulsion was prepared. The emulsion was subcutaneously administered to a rabbit for immunization. After two weeks of immunization, each of the four present immunogens (250 µg) and incomplete Freund's adjuvant (250 µL) were mixed, whereby an emulsion was prepared, and the immunized rabbit was boosted twice with each of the emulsions (subcutaneously and intraperitoneally). Increase in level of specific antibody was determined as follows: One week after the first immunization, blood was drawn from the ear vein and allowed to stand for two hours at room temperature. Subsequently, serum was separated from the blood and subjected to solid-phase immunoassay. One week after the final immunization, whole blood was taken from the heart of the rabbit. The blood was allowed to stand for two hours at room temperature, followed by centrifugation, whereby four antisera (polyclonal antibodies: corresponding to the present antibodies 1A, 1B, 2A, and 2B) were obtained.

### 2) Production of monoclonal antibody

The present immunogen (100 µg), which had been obtained in the form of a complex of the antigen peptide 3 and a hapten (KLH), and complete Freund's adjuvant (100 µL) were mixed, to thereby prepare an emulsion, and the emulsion was subcutaneously administered to a mouse for immunization. After two weeks of the immunization, the complex (50 µg) and incomplete Freund's adjuvant (50 µL) were mixed, to thereby prepare an emulsion, and the mouse was boosted three times with the emulsion (subcutaneously and intraperitoneally). Increase in level of specific antibody in mouse serum was determined as follows: One week after the first immunization, blood was drawn from the tail and allowed to stand for two hours at room temperature. Subsequently, serum was separated from the blood and subjected to solid phase immunoassay. Three days from the final immunization, the spleen was removed from the mouse, and immunocytes contained in the spleen were extracted. In the presence of polyethylene glycol, the immunocytes were subjected to cell fusion with myeloma cells (SP2/0-Ag14), to thereby prepare hybridomas. Monoclonal-antibody-producing hybridomas were selected by confirming the presence of antibodies produced by the hybridomas through solid phase immunoassay. As a result, two monoclonal antibody-producing hybridomas were found to have been obtained. From a culture supernatant of the monoclonal antibody-producing hybridoma, a monoclonal antibody (corresponding to the present antibody 3A) was obtained through a conventional method.

### Specificity of the present antibody

### 1) Specificity of polyclonal antibody

In order to verify specificity of the polyclonal antibodies of the present invention against NV, the four antisera obtained as described above were subjected to the following procedure.

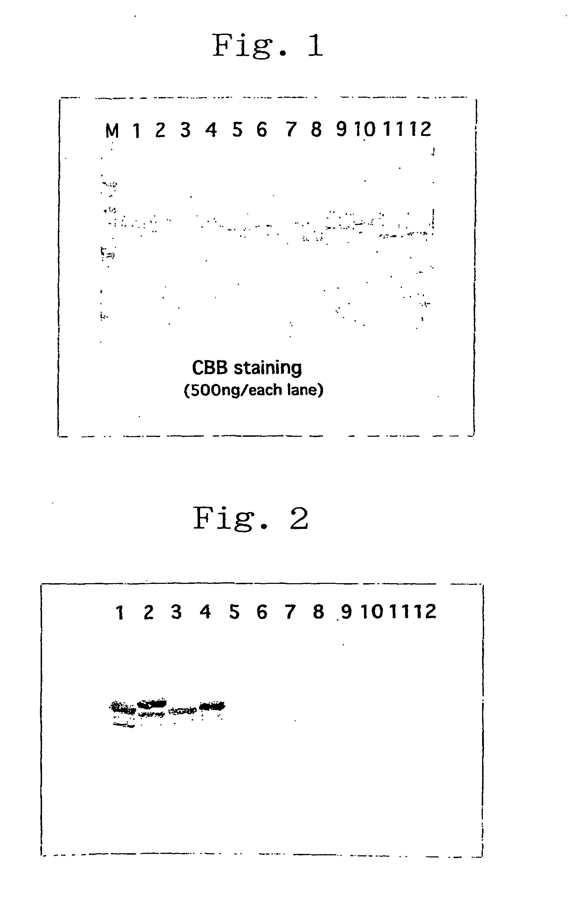

Virus-like particle (VLP) samples of NV (500 ng; G1: 4 strains, G2: 8 strains) were prepared in accordance with a method described by Kobayashi *et al.* (Kobayashi S. *et al.,* Journal of Medical Biology 62: 233-238 (2000), Kobayashi S. *et al.,* Microbiol. Immunol. 44: 687-693 (2000)) [G1-1, G1-2, G1-3, CV (GenBank Accession No. AB022679); these fall under NV(G1)] [G2-1, G2-2, G2-3, G2-4, G2-5, G2-6, G2-7, U201 (GenBank Accession No. AB039782); these fall under NV(G2)]. The gene coding for ORF2 of each VLP sample, originating from stool specimen in which NV particles had been identified under an electron microscope, was subjected to cloning. Each VLP sample was dissolved in SDS, followed by reduction with mercaptoethanol and SDS polyacrylamide gel electrophoresis by use of a mini-slab gel. As a result of electrophoresis, bands of each VLP sample migrated to the locations as expected [Fig. 1 shows the results of CBB staining. A low range marker (Bio-Rad Laboratories, Inc.) was employed as a size marker (M), and the bands indicate, from top to bottom, 97, 66, 46, and 31 kDa].

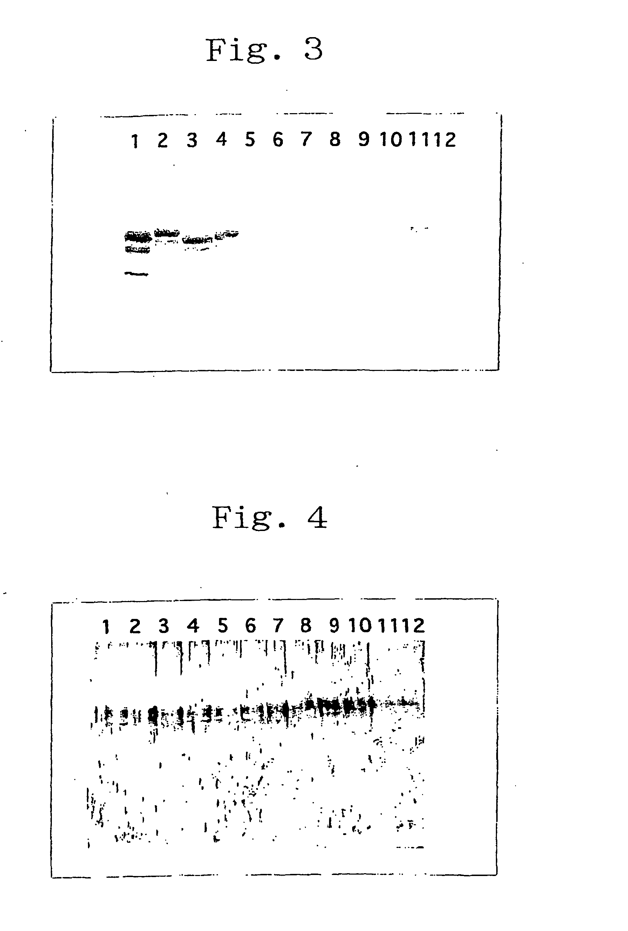

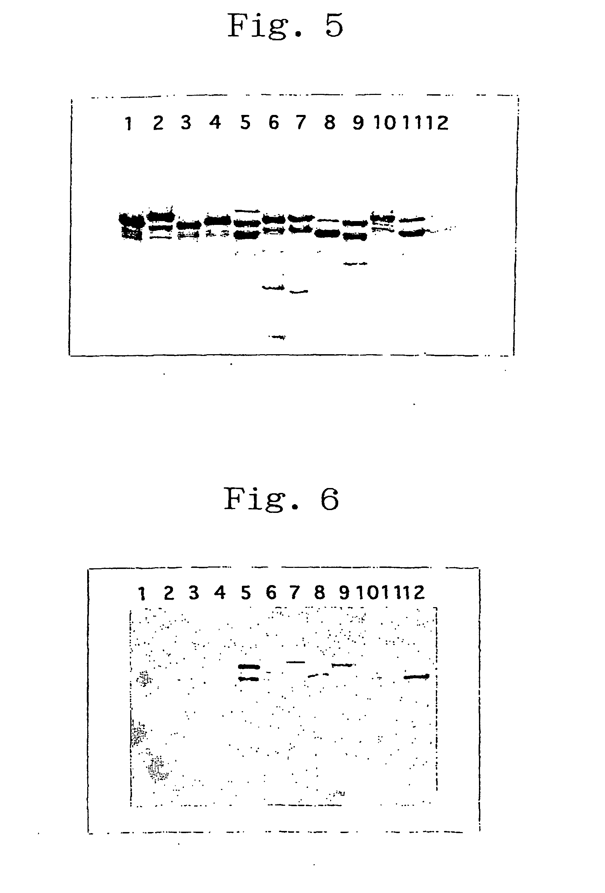

After electrophoresis, in the presence of 20% methanol, the surface of electrophoresis was transferred to a polyvinylidene difluoride membrane with carbonate buffer. The membrane was blocked with 5% skim milk and subjected to reaction, for two hours, with each of the antisera diluted to 3,000-fold. Washing was performed three times with TBST [50mM Tris-HCl (pH 7.5) supplemented with 500mM NaCl and 0.1% Tween 20] for removal of excessive antibodies, and the membrane was subjected to reaction with a secondary antibody [AP-conjugated affinipure F(ab')2 fragment Goat Anti-Rabbit IgG (H+L)] for one hour. Moreover, washing was performed three times with TBST, and the membrane was subjected to reaction with AP Color Reagent (Bio-Rad Laboratories, Inc.) in accordance with the manufacturer's protocol. Subsequently, signals were exposed to a film, to thereby detect the VLPs of NV. The antisera corresponding to the present antibodies 1A and 1B showed specific binding to VLPs of NV(G1) (See Figs. 2 and 3). Here, Figs. 2 to 5 show the results of Western blotting, which indicate binding abilities of the present antibodies against VLPs. The antisera corresponding to the present antibodies 2A and 2B showed specific binding to VLPs of NV(G1) and NV(G2) (See Figs. 4 and 5).

When supernatant concentrates of suspensions of 10% stool specimens of diarrhea patients infected with viruses other than NVs (Rotavirus, Poliovirus, or Adenovirus; including diarrhea caused by vaccination) in sterilized water were employed as negative controls, bands indicating the presence of NVs were not observed.

The results indicate that, when a polyclonal antibody is prepared by use of an immunogen containing an antigen peptide composed of 4 to 11 continuous amino acid residues that fall within the amino acid sequence of SEQ ID NO: 1 or an immunogen containing an antigen peptide composed of 4 to 12 continuous amino acid residues that fall within the amino acid sequence of SEQ ID NO: 2, a polyclonal antibody which binds specifically to NV(G1) (i.e., which binds to NV(G1), but doesn't bind to NV(G2)) can be obtained. It would be apparent that, in the case of a monoclonal antibody, similar results can be obtained.

The results also indicate that, when a polyclonal antibody is prepared by use of an immunogen containing an antigen peptide composed of 4 to 9 continuous amino acid residues that fall within the amino acid sequence of SEQ ID NO: 3 or an immunogen containing an antigen peptide composed of 4 to 11 continuous amino acid residues that fall within the amino acid sequence of SEQ ID NO: 4, a polyclonal antibody which binds to both NV(G1) and NV(G2) can be obtained.

### 2) Specificity of monoclonal antibody

The thus-obtained VLPs were dissolved in SDS, followed by reduction with mercaptoethanol and SDS polyacrylamide gel electrophoresis by use of a mini-slab gel. After electrophoresis, in the presence of 20% methanol, VLPs were transferred to a polyvinylidene difluoride membrane by use of carbonate buffer. The membrane was blocked with 5% skim milk and subjected to reaction, for two hours, with the monoclonal antibody obtained above and diluted to 3,000-fold. Washing was performed three times with TBST (50mM Tris-HCl (pH 7.5) supplemented with 500mM NaCl and 0.1% Tween 20) for removal of excessive antibodies, and the membrane was subjected to reaction with a secondary antibody (AP-conjugated affinipure F(ab')2 fragment Goat Anti-Mouse IgG (H+L)) for one hour. Thereafter, washing was performed three times with TBST, and the membrane was subjected to reaction with AP Color Reagent (Bio-Rad Laboratories, Inc.) in accordance with the manufacturer's protocol. Subsequently, signals were exposed onto a film, whereby VLPs of NV were detected. Western blotting revealed that the monoclonal antibody obtained by immunization of a mouse with an antigen peptide having an amino acid sequence of SEQ ID NO: 3 has ability to bind to capsid protein of NV(G2); that is, the monoclonal antibody has ability to bind to capsid protein of NV(G2) but has no ability to bind to capsid protein of NV(G1) (see Fig. 6).

In the case of a monoclonal antibody produced by another monoclonal-antibody-producing hybridoma, similar results were obtained.

The results indicate that, when a monoclonal antibody is prepared by use of an immunogen containing an antigen peptide composed of 4 to 9 continuous amino acid residues that fall within the amino acid sequence of SEQ ID NO: 3, a monoclonal antibody which binds specifically to NV(G2) can be obtained at high incidence. Also, when a monoclonal antibody is prepared by use of an immunogen containing an antigen peptide composed of 4 to 11 continuous amino acid residues that fall within the amino acid sequence of SEQ ID NO: 4, a monoclonal antibody which binds specifically to NV(G2) can be obtained high incidence.

### The present detection method, etc.

### 1) Preparation of sample

As described above, the present detection method making use of the present antibodies can be employed for detecting virus in watery sources (such as stool, vomitus, river water, seawater, and sewage), foodstuffs, cooking utensils, etc. In the case where one of the watery sources is used as a sample, an undiluted sample, a diluted sample solution diluted with water or an appropriate buffer (e.g., phosphate buffer), or a sample concentrate which is concentrated through centrifugation or filtration is preferably employed.

In contrast, when a sample such as a foodstuff or a cooking utensil undergoes virus detection, the smear test of the sample is preferably performed by use of filter paper. During the test, the filter paper is immersed in water or an appropriate buffer (e.g., phosphate buffer), and the supernatant is used as a sample. Alternatively, water or an appropriate buffer (e.g., phosphate buffer) is added to the filter paper, and the paper is ground with a mixer. The mixture is subjected to filtration or centrifugation, and the resultant filtrate or supernatant (undiluted) is employed as a sample. The sample may be used after dilution with water or an appropriate buffer (e.g., phosphate buffer), or after concentration through, for example, centrifugation or filtration.

In order to facilitate recognition of NV antigen epitopes by the present antibodies, an appropriate protein-denaturing agent (10M urea, a surfactant such as 0.1% SDS, 1% Triton X-100, or 0.1% Tween 20, etc.) may be added to the aforementioned sample, or the sample may be subjected to thermal denaturation (100°C).

### 2) NV Detection by sandwich ELISA

(a) Antibody immobilization: Two different antibodies of the present invention, exhibiting the same characteristics against NV, are selected to form a pair [the antibodies are derived from different animals (e.g., a rabbit and a mouse), have specificity to NV(G1), NV(G2), or both NV(G1) and NV(G2), and may be monoclonal or polyclonal]. One antibody of each pair is diluted with phosphate buffer (10 ng/well to 1 µg/well, pH 7.4), and the solution containing the antibody (50 µL/well) is added to a 96-well microplate for ELISA (MaxiSorp, Nunc), followed by incubation for 24 hours at 4°C, to thereby immobilize antibodies to the bottom of each well of the plate. Subsequently, washing is performed four times with phosphate buffer containing 0.1% Tween 20 (300 µL/well), and a blocking reagent (300 µL/well, Block Ace, Dainippon Pharmaceutical Co., Ltd.) is added to the plate, followed by incubation for two hours at room temperature, to thereby block the portions where no antibody has been immobilized. Thereafter, washing is performed four times with phosphate buffer containing 0.1% Tween 20 (300 µL/well).
(b) Each of the samples prepared as described above is brought into contact with the solid phase described in step (a). Specifically, each of the samples is added to the plate (100 µL/well), followed by incubation for one hour at room temperature. Subsequently, washing is performed three times with phosphate buffer containing 0.1% Tween 20 (300 µL/well).
(c) The other antibody of the pair, which has not been used in step (a), is brought into contact with the solid phase prepared in step (a). Specifically, a solution of the antibody in phosphate buffer containing 10% Block Ace is added (100 µL/well), followed by incubation for one hour at room temperature. Subsequently, washing is performed three times with phosphate buffer containing 0.1% Tween 20 (300 µL/well).
(d) An enzyme-labeled antibody against the antibody used in step (c) is brought into contact with the solid phase prepared in step (a). Specifically, a solution of a peroxidase-labeled secondary antibody in phosphate buffer containing 10% Block Ace is added (100 µL/well), followed by incubation for one hour at room temperature. Subsequently, washing is performed three times with phosphate buffer containing 0.1% Tween 20 (300 µL/well).
(e) After addition of a substrate for the enzyme of the labeled antibody used in step (d), color developed from decomposition of the substrate is measured. Specifically, 0.04% 3,3',5,5'-tetramethylbenzidine is added to each well, and 10 minutes later, the reaction is stopped, followed by measurement of the absorbance at 450 nm. The absorbance of a well to which only water, phosphate buffer, or a similar solvent is added in stead of a sample is defined as a background value (negative control). If the absorbance of a well to which a sample is added is higher than the background value, the sample is found to be NV positive.

As described above, when the present antibodies are used in combination, information in relation to the nature of contamination of the sample with NV is obtained, whereby different purposes, such as detection of the presence or absence of NV contamination and typing of NV contamination ((G1) and/or (G2)), can be accomplished.

The present invention provides a kit for performing the above technique and including, as its elements, at least one of the present antibodies, reagents used for detecting NV, etc.

### 3) NV Detection method by latex agglutination

The present antibody which has been purified in advance is mixed with stirring in a suspension of 1% polystyrene latex particles (0.4 µm) in 0.05M glycine buffer (pH 8.0) containing 0.15M NaCl, and the resultant anti-NV-antibody-sensitized latex particles are washed through centrifugation. Subsequently, the aforementioned latex particles are suspended in a solution (pH 8.2, composition: 0.05M glycine, 1% BSA, and 0.1% NaN₃) so as to attain a concentration of 1%, whereby an NV detecting reagent is prepared.

A sample solution (100 µL) is added dropwise to a reaction plate, and the above-described NV detecting reagent (50 µL) is added dropwise in the vicinity of the sample. Thereafter, the sample and reagent are sufficiently mixed, followed by reaction for about three minutes. Subsequently, agglutination on the plate is observed. As a negative control, water, phosphate buffer, or a similar solvent is employed instead of the sample. When agglutination is not observed in wells, the sample is determined as negative, whereas when agglutination is observed, the sample is determined as positive.

As described above, when the present antibodies are used in combination, information in relation to the nature of contamination of the sample with NV is obtained, whereby any of the following objects can be accomplished: detection of the presence or absence of NV pollution, or typing of NV pollution ((G1) and/or (G2)).

The present invention provides a kit for performing the above technique and including, as its elements, at least one of the present antibodies, reagents used for detecting NV, etc.

### Industrial Applicability

As described above, the present invention provides convenient, accurate means for detecting NV.

## Claims

1. An immunogen containing an antigen peptide composed of 4 to 11 continuous amino acid residues that fall within the amino acid sequence of SEQ ID NO: 1.

2. An immunogen containing an antigen peptide composed of 4 to 12 continuous amino acid residues that fall within the amino acid sequence of SEQ ID NO: 2.

3. An immunogen containing an antigen peptide composed of 4 to 9 continuous amino acid residues that fall within the amino acid sequence of SEQ ID NO: 3.

4. An immunogen containing an antigen peptide composed of 4 to 11 continuous amino acid residues that fall within the amino acid sequence of SEQ ID NO: 4.

5. An antibody which takes, as an antigen, an immunogen containing an antigen peptide composed of 4 to 11 continuous amino acid residues that fall within the amino acid sequence of SEQ ID NO: 1 or an immunogen containing an antigen peptide composed of 4 to 12 continuous amino acid residues that fall within the amino acid sequence of SEQ ID NO: 2, and is capable of binding to Norwalk virus (G1) but not binding to Norwalk virus (G2).

6. An antibody which takes, as an antigen, an immunogen containing an antigen peptide composed of 4 to 9 continuous amino acid residues that fall within the amino acid sequence of SEQ ID NO: 3 or an immunogen containing an antigen peptide composed of 4 to 11 continuous amino acid residues that fall within the amino acid sequence of SEQ ID NO: 4, and is capable of binding to both Norwalk virus (G1) and Norwalk virus (G2).

7. A monoclonal antibody which takes, as an antigen, an immunogen containing an antigen peptide composed of 4 to 9 continuous amino acid residues that fall within the amino acid sequence of SEQ ID NO: 3 or an immunogen containing an antigen peptide composed of 4 to 11 continuous amino acid residues that fall within the amino acid sequence of SEQ ID NO: 4, and is capable of binding to Norwalk virus (G2) but not binding to Norwalk virus (G1).

8. A method of detecting a virus, which comprises using an antibody as recited in claim 5 for detection of Norwalk virus (G1) in a specimen.

9. A method of detecting a virus, which comprises using an antibody as recited in claim 6 for detection of Norwalk virus (G1) and/or Norwalk virus (G2) in a specimen.

10. A method of detecting a virus, which comprises using a monoclonal antibody as recited in claim 7 for detection of Norwalk virus (G2) in a specimen.

11. A method of detecting a virus, which comprises using in combination two or more species of antibodies as recited in any of claims 5 to 7 for detection of Norwalk virus in a specimen.

12. A virus detection kit for performing a virus detection method as recited in claim 8, the kit containing as an element thereof an antibody as recited in claim 5.

13. A virus detection kit for performing a virus detection method as recited in claim 9, the kit containing as an element thereof an antibody as recited in claim 6.

14. A virus detection kit for performing a virus detection method as recited in claim 10, the kit containing as an element thereof a monoclonal antibody as recited in claim 7.

15. A virus detection kit for performing a virus detection method as recited in claim 11, the kit containing as elements thereof two or more species of antibodies as recited in any of claims 5 to 7.
